# EUROPÄISCHE PATENTANMELDUNG

(11) **EP 4 187 928 A1**
(43) Veröffentlichungstag der Anmeldung: **31.05.2023**
(21) Anmeldenummer: 22209274.4
(22) Anmeldetag: 24.11.2022
(51) Int. Cl.: H04R 25/00, H04R 1/10

(54) **VERFAHREN ZUM BETRIEB EINES HÖRINSTRUMENTES**

(30) Priorität: 25.11.2021 DE 102021213300
(71) Anmelder: Sivantos Pte. Ltd., Singapore 539775 (SG)
(72) Erfinder: SERMAN, Maja, 91058 Erlangen (DE); WILSON, Cecil, 91058 Erlangen (DE); GIESE, Ulrich, 91058 Erlangen (DE)
(74) Vertreter: FDST Patentanwälte

(57) **Zusammenfassung**

Die Erfindung nennt ein Verfahren zum Betrieb eines Hörinstrumentes (2), wobei des Hörinstrumentes (2) mittels eines Sensors (22) ein biometrischer Parameter (42) eines Trägers des Hörinstrumentes (2) erfasst wird und/oder mittels wenigstens eines Mikrofons (8) des Hörinstrumentes (2) aus einem Umgebungsschall (14) ein elektrisches Eingangssignal (16) erzeugt wird, und anhand einer Analyse des Eingangssignals (16) das Vorliegen einer bestimmten akustischen Umgebungssituation (48) aus einer Mehrzahl an unterschiedlichen akustischen Umgebungssituationen erkannt wird, anhand des erfassten biometrischen Parameters (42) bzw. der erkannten akustischen Umgebungssituation (48) auf ein kritisches Stressniveau (CSL) des Trägers geschlossen wird, und daraufhin abhängig vom so erschlossenen kritischen Stressniveau (CLS) ein Parameter (54) einer Signalverarbeitung des Hörinstrumentes (2) auf einen vorab festgelegten Parameterwert (52) eingestellt wird.

## Beschreibung

Die Erfindung betrifft ein Verfahren zum Betrieb eines Hörinstrumentes, und weiter ein Hörsystem mit einem Hörinstrument.

Hörschwächen können vielfältige Ursachen haben. So kann bei einem sog. Schallleitungshörverlust einerseits eine im Wesentlichen mechanische Hemmung des Außenohrs bzw. der Organe des Mittelohrs vorliegen, sodass ein im Gehörgang einer Person eindringender Schall nicht ausreichend an die Nervenzellen des Innenohrs weitergeleitet wird. Andererseits können bei einem sog. sensorineuralen Hörverlust die besagten Nervenzellen oder auch der Hörnerv Schädigungen aufweisen. Kombinationen der beiden genannten Formen des Hörverlustes sind auch möglich.

Insbesondere bei der sensorineuralen Form kann infolge der in den Nerven bedingten Ursache des Hörverlustes je nach dessen Grad auch ein allgemeiner Zustand bzw. ein allgemeines Wohlbefinden einer hörgeschwächten Person einen Einfluss darauf haben, in wie weit die Hörschwäche stärker oder schwächer zum Tragen kommt.

Umgekehrt kann bei einem Hörgeschädigten das allgemeine Wohlbefinden beeinträchtigt werden, wenn eine besonders hohe Höranstrengung vorgenommen werden muss, um z.B. einen Gesprächspartner überhaupt verstehen zu können.

Der Erfindung liegt daher die Aufgabe zugrunde, ein Verfahren anzugeben, durch welches sich beim Betrieb eines Hörinstruments in möglichst individuell aussagekräftiger Weise ein allgemeines Wohlbefinden eines Trägers des Hörinstrumentes berücksichtigen lässt. Der Erfindung liegt weiter die Aufgabe zugrunde, ein Hörsystem anzugeben, welches ein Hörinstrument umfasst und mit welchem die genannte Berücksichtigung des allgemeinen Wohlbefindens des Trägers ermöglicht wird.

Die erstgenannte Aufgabe wird erfindungsgemäß gelöst durch ein Verfahren zum Betrieb eines Hörinstrumentes, wobei des Hörinstrumentes mittels eines Sensors, insbesondere eines Sensors des Hörinstrumentes, ein biometrischer Parameter eines Trägers des Hörinstrumentes erfasst wird und/oder mittels wenigstens eines Mikrofons des Hörinstrumentes aus einem Umgebungsschall ein elektrisches Eingangssignal erzeugt wird, und anhand einer Analyse des Eingangssignals das Vorliegen einer bestimmten akustischen Umgebungssituation aus einer Mehrzahl an unterschiedlichen akustischen Umgebungssituationen erkannt wird, anhand des erfassten biometrischen Parameters bzw. der erkannten akustischen Umgebungssituation auf ein kritisches Stressniveau des Trägers geschlossen wird, und daraufhin abhängig vom so erschlossenen kritischen Stressniveau wenigstens ein Parameter einer Signalverarbeitung des Hörinstrumentes auf einen vorab festgelegten Parameterwert eingestellt wird. Vorteilhafte und teils für sich gesehen erfinderische Ausgestaltungen sind in den Unteransprüchen und in der nachfolgenden Beschreibung dargelegt.

Als ein Hörinstrument ist hierbei generell jedwede Vorrichtung umfasst, welche dazu eingerichtet ist, aus einem elektrischen Signal - welches auch durch ein internes Signal der Vorrichtung gegeben sein kann - ein Schallsignal zu erzeugen und einem Gehör eines Trägers dieser Vorrichtung zuzuführen, also insbesondere ein Kopfhörer (z.B. als "Earplug"), ein Headset, eine Datenbrille mit Lautsprecher, etc. Als ein Hörinstrument ist aber auch ein Hörgerät im engeren Sinne umfasst, also ein Gerät, welches zur Versorgung einer Hörschwäche des Trägers eingerichtet ist (als eine Haupt- oder eine Nebenfunktion), und in welchem dazu ein aus einem Umgebungsschall mittels eines Mikrofons erzeugtes Eingangssignal zu einem Ausgangssignal verarbeitet und dabei insbesondere frequenzbandabhängig verstärkt wird, und ein aus dem Ausganssignal mittels eines Lautsprechers o.ä. erzeugtes Ausgangsschallsignal dazu geeignet ist, die Hörschwäche des Trägers (insbesondere benutzerspezifisch) zumindest teilweise zu kompensieren.

Der wenigstens eine Parameterwert wird dabei bevorzugt so vorab festgelegt, dass eine Verarbeitung eines internen Signals - also z.B. bei einem Hörgerät ein Eingangssignal, ab auch generell jedes Ausgangssignal, welches von einem Lautsprecher o.ä. des Hörinstrumentes in ein Ausgangsschallsignal umzuwandeln ist - mit dem Parameterwert für besagten Parameter der Signalverarbeitung für eine Verringerung des Stressniveaus beim Träger vorgesehen ist, und dazu auch geeignet ist. Dies kann z.B. anhand von psychoakustischen Erfahrungswerten für den Parameter erfolgen, anhand von allgemeinen medizinischen Kenntnissen zur Entstehung und Verminderung von Stress und dem Einfluss von Schall hierauf, oder auch durch eine individuelle Abstimmung des Parameters bei einer Anpassung durch einen Hörgeräteakustiker o.ä.

Der mittels des Sensors erfasste biometrische Parameter ist dabei bevorzugt derart, dass aus dem biometrischen Parameter ein Rückschluss auf ein Stressniveau des Trägers möglich ist, also insbesondere eine Korrelation, bevorzugt eine monotone Relation zwischen dem biometrischen Parameter und dem Stressniveau vorliegt. Ein kritisches Stressniveau kann dabei auch direkt über den biometrischen Parameter definiert werden: Liegt der biometrische Parameter oberhalb eines kritischen Wertes, wird anhand von biomedizinischen Überlegungen darauf geschlossen, dass ein kritisches Stressniveau erreicht wurde.

Insbesondere wird dabei als biometrischer Parameter eine Kenngröße einer kardiovaskulären Aktivität des Trägers erfasst. Eine solche Kenngröße einer kardiovaskulären Aktivität des Trägers kann dabei bevorzugt gegeben sein durch eine Herzfrequenz des Trägers und/oder eine Änderungsrate der Herzfrequenz und/oder einen systolischen und/oder diastolischen Blutdruck des Trägers und/oder eine Dauer einer Anspannungsphase des Herzes. Die genannten Größen weisen allgemein eine besonders hohe Korrelation mit einem subjektiv empfundenen Stressniveau auf.

Die Herzfrequenz bzw. der systolische bzw. diastolische Blutdruck wird dabei bevorzugt mittels einer Photoplethysmographie (PPG) gemessen. Der Sensor ist in diesem Fall durch einen PPG-Sensor gegeben. Die Änderungsrate der Herzfrequenz lässt sich dann unmittelbar aus der Herzfrequenz ableiten. Ein PPG-Sensor lässt sich in einfacher Weise direkt in das Hörinstrument integrieren. Die Herzfrequenz bzw. der systolische bzw. diastolische Blutdruck kann aber insbesondere auch gemessen werden mittels eines Gehörgang-Mikrofons, welches beim bestimmungsgemäßen Tragen des Hörinstrumentes in einen Gehörgang des Trägers gerichtet ist. Das Gehörgang-Mikrofon kann dabei insbesondere angeordnet sein in bzw. an einem Ohrstück, welches der Träger für das bestimmungsgemäße Tragen des Hörinstruments wenigstens teilweise in den Eingang des Gehörgangs einführt. Das Gehörgang-Mikrofon ist bevorzugt dazu eingerichtet, auch weitere Funktionen des Hörinstrumentes zu erfüllen, z.B. im Rahmen einer Unterdrückung einer akustischen Rückkopplung am Hörinstrument und/oder einer Unterdrückung einer Okklusion des Gehörgangs durch das Hörinstrument. Die Änderungsrate der Herzfrequenz kann insbesondere von der Herzfrequenz direkt abgeleitet werden.

Eine akustische Umgebungssituation, die insbesondere auch als Hörsituation bezeichnet werden kann, ist bevorzugt zu verstehen als eine Zusammenfassung von potentiell verschiedenen Einzelereignissen des Umgebungsschalls zu einer Gruppe, wobei für die Zusammenfassung einer oder mehrere akustische Parameter wie z.B. frequenzbandweise absolute und relative Schallpegel, Störgeräuschpegel, Rauschhintergründe etc. bewerte werden, und zwei Einzelereignisse bei einer hinreichenden Ähnlichkeit der bewerteten Parameter in dieselbe Gruppe zusammengefasst werden, also derselben akustischen Umgehungssituation zugeordnet werden. Dies kann z.B. über entsprechende Intervalle für die bewerteten Parameter und/oder eine vektorielle Auflistung der jeweils bewerteten Parameter mit anschließender Bestimmung eines Vektorabstands (z.B. zu einem "Basisvektor" für eine akustische Umgebungssituation) erfolgen.

Mithin wird also durch die besagte Analyse bestimmt, welcher der verschiedenen, vorgegebenen akustischen Umgebungssituationen das durch einen aktuellen Umgebungsschall gegebene Einzelereignis zuzuordnen ist.

Insbesondere kann der Normalbetrieb des Hörinstrumentes ein kontinuierliches Lernen derart umfassen, dass einzelne Wertebereiche für den biometrischen Parameter zum Erfassen des kritischen Stressniveaus, einzelne akustische Umgebungssituationen, welche ein kritisches Stressniveau bedingen können, und/oder einzelne Wertebereiche für den Parameter der Signalverarbeitung zur Verringerung des Stressniveaus für eine zukünftige Verwendung erfasst und "gelernt" werden können.

Die Signalverarbeitung mit einem Parameterwert, welcher zu einer Verringerung des Stressniveaus des Trägers vorgesehen und auch geeignet ist, hat folgenden Hintergrund:
Aus psychologischer Sicht bedeutet Stress meist eine Reaktion auf verschiedene Ereignisse, welche sich aus der Beziehung einer Person zu ihrer unmittelbaren Umgebung oder ihrem Umfeld ergeben können. Stress kann dabei akut oder auch chronisch sein, wobei es neben der üblichen Wortbedeutung des "negativen" Stress ("Distress") auch positiven "Eustress" geben kann, und das Auftreten jeweils von den genauen Umständen abhängt.

Die Fähigkeit des menschlichen Gehirns, die Empfindlichkeit gegenüber sensorischen Reizen, welche ggf. irrelevant oder auch stress erzeugend sein können, zu verändern oder auch zu unterdrücken, wird üblicherweise als sensorisches Gating bezeichnet. Einerseits kann ein von einem Hörinstrument erzeugtes Ausgangsschallsignal grundsätzlich mit besagtem sensorischen Gating interferieren, wodurch die körpereigene Unterdrückung von Stress beeinträchtigt werden kann. Andererseits kann eine Hörschwäche infolge einer erhöhten Höranstrengung bei einem Hörgeschädigten zu Stress führen, ebenso wie ein unbequemes Tragen eines Hörinstruments, z.B. bei Drücken oder gar Schmerzen.

Gerade im Fall einer Hörschwäche scheint somit eine bidirektionale Beziehung zwischen Stress und der Hörschwäche zu bestehen: Akutes (negative) Stressempfinden können einen Hörverlust weiter verschlechtern; ein Hörverlust kann zu einem für den Betroffenen nicht mehr bewältigbaren Stress führen. Dabei kann auch eine Art zirkuläre Selbstverstärkung der genannten Effekte einsetzen.

Als konkrete Maßnahme zur Verringerung des Stressniveaus beim Träger des Hörinstruments kann über einen entsprechenden Parameter der Signalverarbeitung bevorzugt eine Rauschunterdrückung (direktional oder einkanalig) erhöht werden, eine Hervorhebung von Nutzsignalen (z.B. Sprache mittels sprach-spezifischer Maßnahmen) verstärkt werden, und/oder Pegelspitzen bzw. generell eine maximale Lautstärke verringert werden. Bei den genannten Maßnahmen können generell auch akustische Artefakte auftreten (insbesondere bei der Rauschunterdrückung und der Hervorhebung von Nutzsignalen), jedoch kann im Fall eines kritischen Stressniveaus dessen Reduktion gegenüber einem möglichst realistischen Klangbild vorübergehend priorisiert werden.

Zur Anwendung bzw. als konkrete Maßnahme der Signalverarbeitung kann dabei insbesondere das Eingangssignal auf ein Vorhandensein eines Sprachsignals um Umgebungsschall hin analysiert werden. Beim Vorhandensein eines Sprachsignals wird bevorzugt der wenigstens eine Parameter der Signalverarbeitung derart auf den vorab festgelegten Parameterwert eingestellt, dass eine Richtwirkung eines Richtmikrofons verstärkt wird, und/oder ein Beginn von Sprachsegmenten hervorgehoben wird, und/oder Frequenzbänder mit einem hohen Sprachanteil angehoben werden. Hierdurch wird das Sprachsignal insgesamt hervorgehoben. Bei einer festgestellten Abwesenheit eines Sprachsignals werden bevorzugt eine frequenzbandweise Verstärkung und/oder ein frequenzbandweiser maximaler Ausgangspegel abgesenkt, und/oder eine Einregelzeit einer Kompression verkürzt und/oder eine Abklingzeit einer Kompression verlängert, und/oder eine Anwendungsstärkte einer Rauschunterdrückung und/oder eines Glättungsfilters angehoben werden. Dies verringert allgemein die Belastung durch zu hohe Schallpegel und/oder hohe Pegelspitzen sowie drastische Pegeländerungen.

Günstigerweise kann bei einem kritischen Stressniveau eine Anwendungsstärke und/oder eine Notch-Tiefe eines Tinnitus-Maskierungssignals erhöht werden, bspw. indem für ein Tinnitus-Maskierungssignal die Gesamtlautstärke erhöht wird, eine Schwerpunktsfrequenz abgesenkt wird, und/oder eine Modulationstiefe erhöht wird. Es ist bekannt, dass Tinnitus oftmals einen erheblichen Zusammenhang mit dem Stressniveau der betroffenen Person aufweist, sodass eine verbesserte Maskierung des Tinnitus zur Verringerung des Stressniveaus beitragen kann.

Wird als biometrischer Parameter eine Herzfrequenz und/oder deren Änderungsrate und/oder eine sonstige Kenngröße der kardiovaskulären Aktivität erfasst, so wird bevorzugt mittels eines Beschleunigungssensors und/oder eines Bewegungssensors ein Bewegungszustand des Trägers ermittelt, und für das kritische Stressniveau auch der ermittelte Bewegungszustand des Trägers herangezogen. Somit kann z.B. eine Erhöhung der Herzfrequenz oder des systolischen oder diastolischen Blutdrucks infolge von Bewegung erkannt werden, sodass derartige Erhöhungen nicht einem kritischen Stressniveau zugeordnet werden bzw. starker Bewegungsaktivität ein hinsichtlich des Stressniveaus kritischer Wert für die Herzfrequenz bzw. den betreffenden Blutdruck entsprechend höher angesetzt werden.

Vorteilhafterweise kann dabei zusätzlich zur Kenngröße der kardiovaskulären Aktivität für das kritische Stressniveau auch die erkannte akustische Umgebungssituation und/oder eine Tageszeit herangezogen werden. Die Tageszeit kann dabei insbesondere relevant sein, wenn der Träger regelmäßig eine Einnahme von Medikamenten vornimmt, welche Einfluss auf die Herzfrequenz und/oder einen Blutdruck haben. Eine Veränderung der Herzfrequenz bzw. deren Änderungsrate kann dann entsprechend der Einnahme der Medikation zugerechnet werden, und somit ein hinsichtlich des Stressniveaus kritischer Wert für die Herzfrequenz bzw. einen Blutdruck entsprechend höher angesetzt werden. Eine akustische Umgebungssituation kann z.B. aufgrund allgemeiner Erfahrungswerte entsprechend berücksichtigt werden.

In einer vorteilhaften Ausgestaltung wird anhand des Eingangssignals das Vorliegen einer bestimmten akustischen Umgebungssituation aus der Mehrzahl an unterschiedlichen akustischen Umgebungssituationen erkannt, bei einem Erfassen eines kritischen Stressniveaus des Trägers, insbesondere durch eine Benutzereingabe mittels einer mit dem Hörinstrument verbundenen Hilfsvorrichtung wie z.B. einem Smartphone oder einer Smartwatch, das kritische Stressniveau der bestimmten akustischen Umgebungssituation zugeordnet, und anhand der Analyse des Eingangssignals das Vorliegen der bestimmten akustischen Umgebungssituation erkannt, und anhand der erkannten akustischen Umgebungssituation auf das kritisches Stressniveau des Trägers geschlossen. Dies ist eine weitere Möglichkeit, ein kritisches Stressniveau zu erkennen. Die akustische Umgebungssituation kann dabei als alleiniges Kriterium für das kritische Stressniveau herangezogen werden, oder zusammen mit der Herzfrequenz bzw. deren Änderungsrate jeweils gewichtet werden.

Vorteilhafterweise wird zunächst ein Ruhewert für die Kenngröße der kardiovaskulären Aktivität ermittelt, die erfasste Kenngröße der kardiovaskulären Aktivität mit dem entsprechenden Ruhewert verglichen, und hieraus auf das kritisches Stressniveau des Trägers geschlossen. Bei einem besonders hohen oder niedrigen Ruhewert bspw. für einen Blutdruck, die Herzfrequenz oder deren Änderungsrate ist davon auszugehen, dass auch ein Blutdruck, die Herzfrequenz bzw. ihre Änderungsrate für ein kritisches Stressniveau entsprechend erhöht bzw. verringert ist.

Vorteilhafterweise werden dabei zum Ermitteln des Ruhewertes jeweils der Bewegungszustand des Trägers, welcher mittels des Beschleunigungssensors bzw. des Bewegungssensors ermittelt wird, und/oder eine Tageszeit und/oder eine Kenngröße für einen Umgebungsschall, welche anhand einer entsprechenden Analyse des Eingangssignals ermittelt wird, herangezogen. Die Kenngröße für den Umgebungsschall kann dabei insbesondere auf Störgeräusche, eine klassifizierte Hörsituation, einen Sprachanteil, eine eigene Stimme, ein gesamtlautstärke, und/oder einen Frequenzgang des Umgebungsschalls bezogen sein.

Bewegung, z.B. sportliche Betätigung, kann die Herzfrequenz bzw. den Blutdruck erhöhen, und die Anspannungsphase verkürzen. Die Tageszeit kann insbesondere bei Personen mit regelmäßiger Einnahme von Medikamenten (z.B. Blutdrucksenker o.ä.) in Abhängigkeit dieser Einnahme einen Einfluss auf die Herzfrequenz bzw. deren Änderungsrate haben. Laute Geräusche, insbesondere Störgeräusche, aber generell auch Sprachsignale, insbesondere in lauter Umgebung (z.B. "Cocktail-Party"-Hörsituation) und sogar das Hören der eigenen Stimme können allgemein das Stressniveau erhöhen. Die in entsprechenden Zeitsequenzen ermittelten Werte für die Kenngröße der kardiovaskulären Aktivität werden beim Ermitteln des jeweiligen Ruhewertes nicht bzw. nur in verringertem Umfang herangezogen.

In einer vorteilhaften Ausgestaltung wird anhand einer durch den Träger mittels einer Benutzerschnittstelle vorgenommenen Benutzereingabe , insbesondere über ein mit dem Hörinstrument verbundenes Hilfsgerät wie z.B. ein Smartphone oder eine Smartwatch, ein kritischer Wert für die Kenngröße der kardiovaskulären Aktivität ermittelt wird, welcher einem kritischen Stressniveau des Trägers entspricht, wobei zu einem späteren Zeitpunkt in Abhängigkeit des kritischen Wertes für die Kenngröße der kardiovaskulären Aktivität auf das kritische Stressniveau geschlossen wird, also z.B. wenn ein Blutdruck oder die Herzfrequenz bzw. die Änderungsrate der Herzfrequenz den entsprechenden kritischen Wert erreicht oder überschreitet. Die Benutzerschnittstelle ist hierbei bevorzugt in einer mit dem Hörinstrument datentechnisch verbindbaren Hilfsvorrichtung wie z.B. einem Smartphone o.ä. implementiert. Dies bedeutet insbesondere, dass der Träger durch die Benutzereingabe signalisieren kann, dass bei ihm zum betreffenden Zeitpunkt ein kritisches Stressniveau vorliegt, sodass der entsprechende Wert für einen Blutdruck die Herzfrequenz bzw. deren Änderungsrate als Indikator des kritischen Stressniveaus herangezogen werden kann. Die Benutzereingabe kann dabei z.B. über eine entsprechende App im Smartphone erfolgen.

Als weiter vorteilhaft erweist es sich, wenn anhand des Eingangssignals das Vorliegen einer bestimmten akustischen Umgebungssituation aus der Mehrzahl an unterschiedlichen akustischen Umgebungssituationen erkannt wird, ein der bestimmten akustischen Umgebungssituation vorab zugeordnetes Hörprogramm für die Signalverarbeitung des Eingangssignals angewandt wird, welches dem besagten wenigstens einen Parameter einen bestimmten Parameterwert zuweist, anhand einer Benutzereingabe des Trägers eine Bewertung des Hörprogramms durch den Benutzer erfasst wird, bei einem Erfassen eines kritischen Stressniveaus des Trägers im Fall einer negativen Bewertung des Hörprogramms die Gewichtung der negativen Bewertung bei einem Benutzer-assistierten, automatischen Anpassen des Parameterwertes für die bestimmte akustische Umgebungssituation verringert wird, und/oder im Fall einer positiven Bewertung des Hörprogramms aus dem Hörprogramm ein den Parameterwert umfassendes Unterprogramm erzeugt wird, dessen Anwendung für die bestimmte akustische Umgebungssituation bei einem kritischen Stressniveau des Trägers erfolgt.

Dies erlaubt einerseits, negative Bewertungen von Hörprogrammen, welche lediglich oder wesentlich auf einem akuten Stressempfinden des Trägers beruhen, für die Erzeugung des Parameterwertes zur Signalverarbeitung herauszufiltern. Zudem können Hörprogramme, welche bei einem kritischen Stressniveau eine positive Bewertung erhalten haben, bei erneutem kritischen Stressniveau angewandt werden (also bei späteren Anwendungen verwendet werden). Hierdurch wird aus einem betreffenden Hörprogramm ein Unterprogramm definiert, welches bevorzugt beim gleichzeitigen Vorliegen einer akustischen Umgebungssituation ("Hörsituation") und einem ein kritischen Stressniveau Anwendung findet.

Unter einem Hörprogramm ist dabei insbesondere die Gesamtheit von Parametern der Signalverarbeitung zu verstehen, welche in einer bestimmten akustischen Umgebungssituation auf ein Eingangssignal zu Erzeugung eines Ausgangssignals angewandt werden.

Die zweitgenannte Aufgabe wird erfindungsgemäß gelöst durch ein Hörsystem, umfassend ein Hörinstrument, einen Sensor zum Erfassen eines biometrischen Parameters eines Trägers des Hörinstrumentes, welcher Aufschluss über ein kritisches Stressniveau des Trägers gibt, und eine Steuereinrichtung, wobei die Steuereinrichtung dazu eingerichtet ist, das Hörinstrument in Abhängigkeit des biometrischen Parameters für das kritische Stressniveau gemäß dem Verfahren nach einem der vorhergehenden Ansprüche zu betreiben.

Das Hörsystem kann dabei insbesondere ein mit dem Hörinstrument verbindbares Hilfsgerät (z.B. ein Smartphone, eine Smartwatch o.ä.) umfassen. Das Hilfsgerät kann in diesem Fall die Steuereinrichtung und oder den bzw. wenigstens einen Sensor zum Erfassen des besagten biometrischen Parameters umfassen sowie insbesondere Mittel zum Erfassen einer Benutzereingabe umfassen. Das Hörinstrument ist dabei bevorzugt ausgestaltet als ein Hörgerät. Die Steuereinrichtung kann jedoch auch durch eine Signalverarbeitungseinheit des Hörinstrumentes gegeben sein. Die Durchführung der für das Verfahren erforderlichen Berechnungsschritte können auch teilweise auf einer Prozessoreinheit des Hilfsgerätes und teilweise auf der Signalverarbeitungseinrichtung des Hörinstrumentes erfolgen. Im letztgenannten Fall verteilt sich die Steuereinrichtung auf das Hörinstrument und das Hilfsgerät.

Das erfindungsgemäße Hörsystem teilt die Vorzüge des erfindungsgemäßen Verfahrens. Die für das Verfahren und für seine Weiterbildungen angegebenen Vorteile können dabei, mutatis mutandis, sinngemäß auf das Hörsystem übertragen werden.

Nachfolgend wird ein Ausführungsbeispiel der Erfindung anhand von Zeichnungen näher erläutert. Hierbei zeigen jeweils schematisch:
- Fig. 1: in einem Blockschaltbild ein Hörsystem mit einem Hörgerät und einem Smartphone, welches dazu eingerichtet ist, ein kritisches Stressniveau eines Benutzers zu erfassen,
- Fig. 2: in einem Blockdiagramm ein Verfahren zum Erkennen eines kritischen Stressniveaus eines Trägers eines Hörgerätes mithilfe des Hörgerätes,
- Fig. 3: in einem Blockdiagramm eine alternative Ausgestaltung des Verfahrens nach Fig. 2,
- Fig. 4: in einem Blockdiagramm eine weitere Alternative des Verfahrens nach Fig. 2, und
- Fig. 5: in einem Blockschaltbild ein Hörsystem mit einem zu Fig. 1 alternativen Hörgerät.

Einander entsprechende Teile und Größen sind in allen Figuren jeweils mit denselben Bezugszeichen versehen.

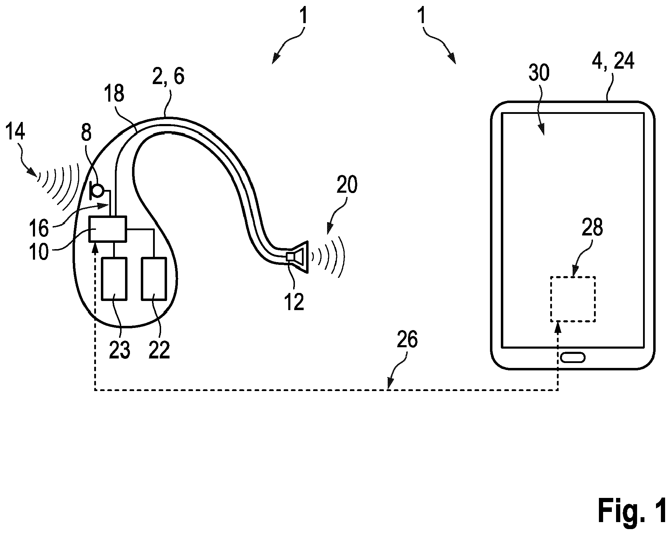

In Figur 1 ist schematisch in einem Blockschaltbild ein Hörsystem 1 dargestellt, welches ein Hörinstrument 2 und ein Hilfsgerät 4 umfasst. Das Hörinstrument 2 ist vorliegend ausgestaltet als ein Hörgerät 6, und weist entsprechend ein Mikrofon 8, eine mit dem Mikrofon 8 verbundene Signalverarbeitungseinrichtung 10 sowie einen mit der Signalverarbeitungseinrichtung 10 verbundenen Lautsprecher 12 auf. Das Hörgerät 6 kann dabei auch noch weitere, in Figur 1 nicht dargestellte Mikrofone aufweisen, welche entsprechend mit der Signalverarbeitungseinrichtung 10 verbunden sind. Das Mikrofon 8 ist dazu eingerichtet, aus einem Umgebungsschall 14 ein Eingangssignal 16 zu erzeugen. Das Eingangssignal 16 (und ggf. weitere Eingangssignale von nicht dargestellten Mikrofonen) wird in der Signalverarbeitungseinrichtung 10 zu einem Ausgangssignal 18 verarbeitet, welches durch den Lautsprecher 12 in ein Ausgangsschallsignal 20 umgewandelt wird. Bei der Verarbeitung des Eingangssignals 16 in der Signalverarbeitungseinrichtung 10 kann insbesondere ein Hörverlust eines Trägers des Hörgerätes 6 dahingehend berücksichtigt werden, dass durch eine frequenzbandabhängige Verstärkung und/oder Kompression des Eingangssignals 16 der Hörverlust zumindest teilweise kompensiert wird.

Weiter weist das Hörgerät 6 einen PPG-Sensor 22 auf, welcher ebenfalls mit der Signalverarbeitungseinrichtung 10 verbunden ist. Der PPG-Sensor 22 ist dazu eingerichtet, eine Herzfrequenz des Trägers zu messen, sodass anhand der gemessenen Herzfrequenz in der Signalverarbeitungseinrichtung 10 auch eine Änderungsrate der Herzfrequenz ermittelt werden kann. Das Hörgerät 6 weist überdies einen Beschleunigungssensor 23 auf, welcher ebenfalls mit der Signalverarbeitungseinrichtung 10 verbunden ist, und mittels dessen eine Bewegungsänderung des Trägers des Hörgerätes 6 erfasst werden kann. Durch eine Integration der Beschleunigung in der Signalverarbeitungseinrichtung 10 kann somit auch eine Bewegung des Trägers erfasst werden.

In noch zu beschreibender Weise wird anhand der erfassten Herzfrequenz bzw. deren Änderungsrate wenigstens eine Parameter der in der Signalverarbeitungseinrichtung 10 erfolgenden Signalverarbeitung des Eingangssignals 16 derart angepasst, dass das entsprechende Ausgangsschallsignal 20 dazu geeignet ist, ein Stressniveau des Trägers des Hörgerätes 6 zu reduzieren. Hierfür wird insbesondere das vorliegend als ein Smartphone 24 ausgestaltete Hilfsgerät 4 verwendet, welches über eine Bluetooth-Verbindung 26 mit dem Hörgerät 6 verbindbar ist. Dazu weisen das Hörgerät 6 und das Smartphone 24 jeweils entsprechend eingerichtete, in Figur 1 nicht näher dargestellte Sende- und Empfangsvorrichtungen (zum Beispiel Antennen) zum Aufbau besagter Bluetooth-Verbindung 26 auf. Das Smartphone 24 weist zudem für nachfolgend beschriebene Verarbeitungsschritte eine Prozessoreinheit 28 auf, sowie einen Touchscreen 30, mittels dessen über eine entsprechende Applikation Benutzereingaben durch den Träger des Hörgerätes 6 bzw. des Hörsystems 1 vorgenommen werden können.

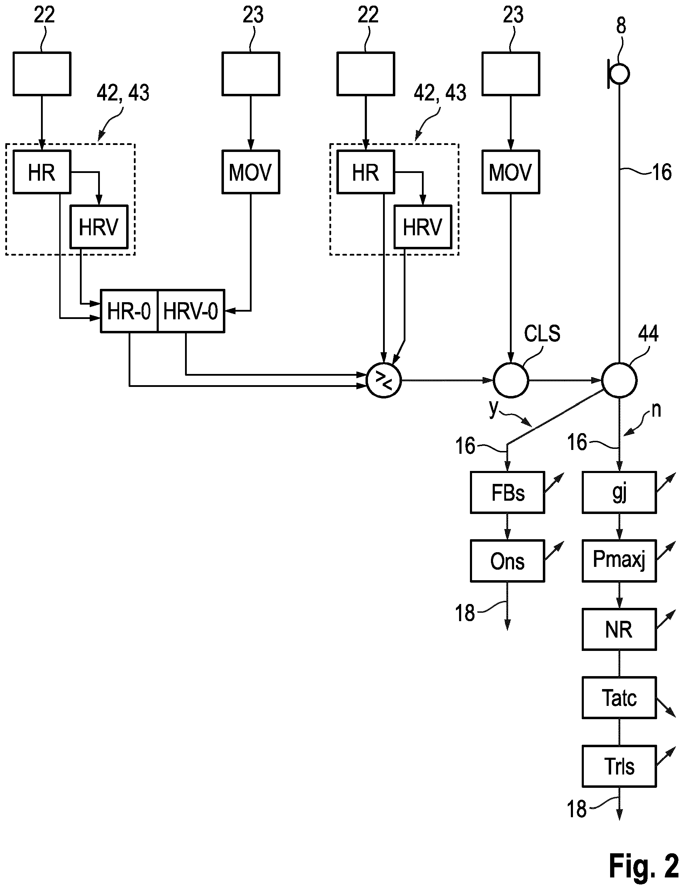

In Figur 2 ist schematisch in einem Blockdiagramm der Ablauf eines Verfahrens dargestellt, durch welches das Hörinstrument 2 nach Figur 1 in Abhängigkeit eines kritischen Stressniveaus des Trägers des Hörinstruments 2 betrieben werden kann. Zunächst werden im laufenden Betrieb des Hörinstruments in nachfolgend beschriebener Weise Zusammenhänge zwischen dem Stressempfinden des Trägers des Höheinstrumentes 2 nach Figur 1, dafür aussagekräftigen biometrischen Parametern und Parametern der Signalverarbeitung, welche das das Stressniveau beeinflussen können, ermittelt und somit "erlernt" (dargestellt in der linken Bildhälfte). Besagte "erlerne" Zusammenhänge hinsichtlich der biometrischen Parameter werden zu einem späteren Zeitpunkt (dargestellt in der rechten Bildhälfte) zum Erkennen einer akut vorliegenden Stresssituation für den Träger des Hörinstrumentes 2 verwendet, sodass die dafür vorgesehenen Parameter der Signalverarbeitung entsprechend auf das Eingangssignal 16 angewandt werden, um mittels des hieraus erzeugten Ausgangsschallsignals 20 das Stressniveau zu reduzieren.

Es werden nun mithilfe des PPG-Sensors 22 biometrische Parameter 42 des Trägers des Hörgerätes 6 ermittelt. Diese biometrischen Parameter 42 sind vorliegend gegeben durch Kenngrößen 43 einer kardiovaskulären Aktivität des Trägers, und umfassen eine Herzfrequenz HR des Trägers des Hörgerätes 6, welche durch den PPG-Sensor 22 erfasst wird, sowie eine Änderungsrate HRV der Herzfrequenz HR, welche in der Signalverarbeitungseinrichtung 10 aus der Herzfrequenz HR ermittelt wird. Weiter können die Kenngrößen 43 einer kardiovaskulären Aktivität bspw. auch einen systolischen und/oder diastolischen Blutdruck und/oder eine Dauer einer Herz-Anspannungsphase umfassen. Anhand des Beschleunigungssensors 23 wird eine Bewegungsaktivität MOV des Trägers des Hörgerätes 6 ermittelt. Aus der Herzfrequenz HR und deren Änderungsrate HRV werden nun durch einen Abgleich mit der ermittelten Bewegungsaktivität MOV jeweils Ruhewerte HR-0 bzw. HRV-0 ermittelt, indem die Werte der Herzfrequenz HR und deren Änderungsrate HRV nur in denjenigen Zeitsequenzen herangezogen werden, in welchen keine nennenswerte Bewegungsaktivität MOV vorliegt. Wie bereits weiter oben beschrieben, kann hierfür zudem auch eine Tageszeit (insbesondere bei der regelmäßigen Einnahme von Medikamenten) und/oder ein Störgeräuschpegel bzw. -hintergrund für das Ermitteln der Ruhewerte HR-0, HRV-0 herangezogen werden.

Zu einem späteren Zeitpunkt (rechte Bildhälfte) wird nun ebenfalls durch den PPG-Sensor 22 die Herzfrequenz HR des Trägers gemessen, und entsprechend deren Änderungsrate HRV ermittelt. Diese beiden Größen werden nun mit den entsprechenden Ruhewerten HR-0, HRV-0 verglichen, welche zuvor ermittelt wurden. Im Fall einer als kritisch bewerteten Abweichung (z.B. eines der beiden genannten biometrischen Parameter 42, oder einer vorgegebenen kartesischen Abweichung für den Vektor [HR, HRV]^{T} vom entsprechenden Vektor der Ruhewerte o.ä.) erfolgt zunächst noch eine Überprüfung, ob anhand des Beschleunigungssensors 23 eine aktuell vorliegende Bewegungsaktivität MOV ermittelt wurde (zusätzlich kann auch noch ein Abgleich hinsichtlich der Tageszeit erfolgen, s.o.). Ist dies nicht der Fall, so wird bei einer hinreichend hohen, also als kritisch bewerteten Abweichung der Herzfrequenz HR bzw. deren Änderungsrate HRV von ihren entsprechenden Ruhewerten auf ein kritisches Stressniveau CSL geschlossen.

In diesem Fall, also wenn ein kritisches Stressniveau vorliegt, so wird das Eingangssignal 16, welches durch das Mikrofon 8 des Hörgerätes 6 aus dem Umgebungsschall 14 zum nun vorliegenden Zeitpunkt erzeugt wird, einer Sprachanalyse 44 unterzogen, welche überprüft, ob im Eingangssignal 16 ein Sprachsignal vorliegt. Ist dies nicht der Fall (Pfad "n"), so werden frequenzbandweise Verstärkungsfaktoren gj sowie frequenzbandweise maximale Ausgangspegel Pmaxj jeweils abgesenkt, sowie eine Rauschunterdrückung NR erhöht. Weiter kann dabei auch eine Einregelzeit Tatc einer Kompression verringert und eine Abklingzeit Trls der Kompression erhöht werden. Durch die genannten Maßnahmen wird das Eingangssignal 16 derart zum Ausgangssignal 18 verarbeitet, dass das durch den Lautsprecher 12 entsprechend erzeugte Ausgangsschallsignal 20 dazu geeignet ist, das Stressniveau beim Träger zu reduzieren. Insbesondere wird dabei die Lärmbelastung für den Träger des Hörgerätes 6 durch die Absenkung der frequenzbandweisen Verstärkungsfaktoren gj verringert, und die Unbehaglichkeitsschwelle, welche sich bei hohem Stressempfinden psychologisch bedingt verringern kann, durch das Absenken der maximalen Ausgangspegel Pmaxj zusätzlich gemieden. Auch wenn durch eine stärkere Anwendung der Rauschunterdrückung NR und/oder eine "schärfere" Anwendung der Kompression ggf. leichter akustische Artefakte auftreten können, ist dies im vorliegenden Fall zur Verringerung des Stressniveaus des Trägers vorübergehend vorzuziehen.

Falls im Eingangssignal 16 ein Sprachsignal vorliegt (Pfad "y" der Sprachanalyse 44), so werden diejenigen Frequenzbänder FBs im Eingangssignal 16, welche einen hohen Sprachanteil aufweisen, relativ angehoben. Weiter wird jeweils der Beginn Ons von Sprachsegmenten hervorgehoben. Im Fall mehrerer Eingangssignale (nicht dargestellt) des Hörgerätes 6, welche von der Signalverarbeitungseinrichtung 10 mittels Richtmikrofonie verarbeitet werden, kann auch die Richtwirkung erhöht werden. Die genannten Maßnahmen tragen beim Vorliegen eines Sprachsignals dazu bei, die Sprachverständlichkeit wirksam zu verbessern, und dadurch das Stressniveau des Trägers des Hörgerätes 6 zu verringern (auch wenn dabei ggf. ein realistisches Hörempfinden vorrübergehend beeinträchtigt werden könnte).

Die genannten Maßnahmen, welche jeweils beim Vorliegen oder der Abwesenheit eines Sprachsignals und gleichzeitigem Vorliegen des kritischen Stressniveaus CSL hinsichtlich der Verarbeitung des Eingangssignals 16 getroffen werden, können dabei insbesondere im Rahmen eines konkreten Hörprogramms (welches abhängig von einer akustischen Umgebungssituation ggf. weitere Maßnahmen der Signalverarbeitung umfasst) getroffen werden, welches in einer akustischen Umgebungssituation spezifisch für den Fall eines kritischen Stressniveaus CSL vorgesehen ist. Andererseits können die Maßnahmen auch "ad-hoc" und unabhängig von einem Hörprogramm durchgeführt werden, sodass die Anwendung lediglich in Abhängigkeit des kritischen Stressniveaus CSL und ggf. der Sprachanalyse 44 erfolgt. Generell wird dabei der betreffende Parameter auf einen Parameterwert eingestellt, dass eine Signalverarbeitung des Eingangssignals 16 mit dem Parameterwert für den Parameter zu einer Verringerung des Stressniveaus geeignet ist, also erwartungsgemäß und ohne unvorhersehbare Ausnahmeumstände dazu führen wird.

Wird kein kritisches Stressniveau CSL festgestellt, also etwa, da keine hinreichend hohe Abweichung der Herzfrequenz HR bzw. deren Änderungsrate HRV von den jeweiligen Ruhewerten HR-0, HRV-0 vorliegt, oder weil eine hinreichend hohe Abweichung auf eine hohe Bewegungsaktivität MOV zurückgeführt werden kann, so erfolgt die Verarbeitung des Eingangssignals 16 zum Ausgangssignal 18 bevorzugt lediglich in Abhängigkeit einer anhand des Eingangssignals 16 selbst festgestellten akustischen Umgebungssituation ("Hörsituation"; nicht dargestellt).

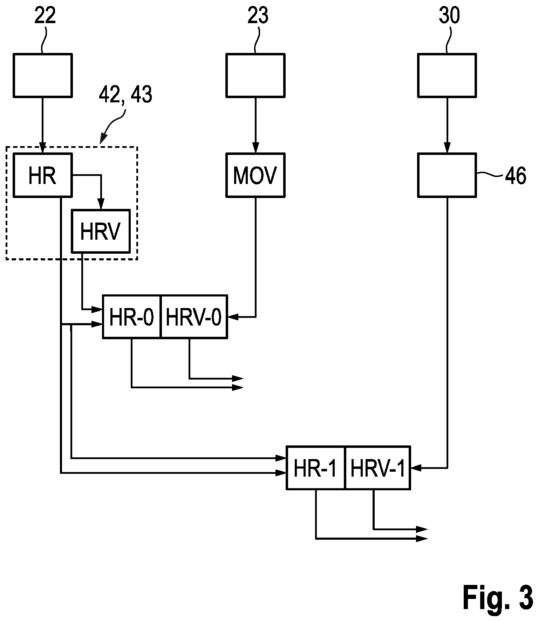

In Figur 3 ist schematisch in einem Blockdiagramm eine alternative Ausgestaltung desjenigen Teils des Verfahrens nach Figur 2 dargestellt, welcher das "Erlernen" des Erkennens der korrekten Werte der biometrischen Parameter 42 für das kritische Stressniveau CSL betrifft (linke Bildhälfte in Figur 2). Wie im Ausführungsbeispiel nach Figur 2 werden als biometrische Parameter 42 und somit als Kenngrößen 43 der kardiovaskulären Aktivität des Trägers die Herzfrequenz HR und hieraus deren Änderungsrate HRV gemessen bzw. ermittelt, um über einen Abgleich mit der Bewegungsaktivität MOV die Ruhewerte HR-0, HRV-0 der genannten biometrischen Parameter 42 zu bestimmen.

Zudem wird eine Benutzereingabe 46 erfasst, welche der Träger des Hörgerätes 6 über den Touchscreen 30 des Smartphones 24 in einer entsprechenden App eingeben kann. Durch die Benutzereingabe 46 signalisiert der Träger dem Hörsystem 1 nach Figur 1, dass für ihn selbst gerade das Stressempfinden unangenehm hoch ist. Hinsichtlich der Herzfrequenz HR und ihrer Änderungsrate HRV bedeutet dies, dass die durch die Benutzereingabe 46 als kritisch ermittelten Werte HR-1 bzw. HRV-1 einem kritischen Stressniveau CSL entsprechen. In der in Figur 3 nicht dargestellten Anwendung (vgl. rechte Bildhälfte in Figur 2) kann nun das kritische Stressniveau CSL erkannt werden, wenn die mittels des PPG-Sensors 22 festgestellte Herzfrequenz HR (bzw. ihre Änderungsrate HRV) hinreichend nahe beim für das kritische Stressniveau CSL ermittelten Wert HR-1 (bzw. HRV-1) oder darüber liegt. Für Werte unterhalb von HR-1 (bzw. HRV-1) kann dabei der Ruhewert HR-0 (bzw. HRV-0) für den als kritisch zu erachtenden Wertebereich herangezogen werden.

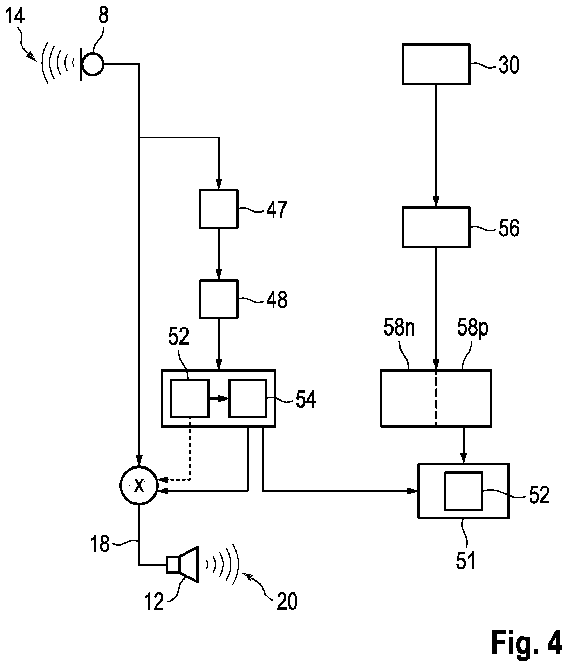

In Figur 4 ist schematisch in einem Blockdiagramm eine weitere Alternative zum Verfahren nach Figur 2 dargestellt. Dabei wird das Eingangssignal 16, welches durch das Mikrofon 8 des Hörgerätes 6 aus dem Umgebungsschall 14 zu einem gegebenen Zeitpunkt erzeugt wird, einer Analyse 47 unterzogen, in welcher eine im Umgebungsschall 14 repräsentierte akustische Umgebungssituation 48 erkannt wird. Es wird nun ein der erkannten akustischen Umgebungssituation 48 vorab zugeordnetes Hörprogramm 50 ausgewählt, welches bestimmte Parameterwerte 52 für einzelne Parameter 54 der Signalverarbeitung (wie z.B. die in der Beschreibung von Figur 2 genannten Parameter, welche auf das Eingangssignal 16 angewandt werden) festlegt. Das Eingangssignal 16 wird nun gemäß den durch das Hörprogramm 50 festgelegten Parameterwerten 52 für die Parameter 54 verarbeitet, und das daraus resultierende Ausgangssignal 18 durch den Lautsprecher 12 in das Ausgangsschallsignal 20 umgewandelt.

Der Träger (nicht dargestellt) des Hörgerätes 6 "hört" nun dieses Ausgangsschallsignal, und kann dabei über eine Benutzereingabe 56 (auf vergleichbarem Weg wie die Benutzereingabe 46 nach Figur 3) eine positive oder negative Bewertung 58p bzw. 58n des Hörprogramms 50 vornehmen. Zudem kann der Träger über eine Benutzereingabe 46 dem Hörsystem 1 ein subjektiv empfundenes kritisches Stressniveau CSL melden. Im Fall, dass eine solche Meldung eines kritischen Stressniveaus CSL durch die Benutzereingabe 46 vorliegt, kann die negative Bewertung 58n des Hörprogramms 50 verfahrensgemäß zu einer geringeren Gewichtung der zugehörigen Parameterwerte 52 für eine abschließende Anpassung des Hörprogramms 50 oder auch zu einer Änderung der Parameterwerte 52 beim nächsten Auftreten der akustischen Umgebungssituation 48 führen. Liegt jedoch stattdessen eine positive Bewertung 58p des Hörprogramms 50 vor, so kann anhand der entsprechend verwendeten Parameterwerte 52 auch ein Unterprogramm 51 generiert werden, welches für eine spätere Anwendung beim gleichzeitigen Vorliegen der akustischen Umgebungssituation 48 (welcher das Hörprogramm 50 zugeordnet ist) und des kritischen Stressniveaus CLS (bevorzugt anhand der biometrischen Parameter 42 zu ermitteln) vorgesehen ist.

Analog zu den Ausführungsbeispielen nach Figur 2 und Figur 3 können zusätzlich als biometrische Parameter 42 die Herzfrequenz HR und hieraus deren Änderungsrate HRV sowie ggf. auch weitere als die in Figur 2 gezeigten Kenngrößen 43 der kardiovaskulären Aktivität gemessen bzw. ermittelt werden (nicht dargestellt). Des Weiteren kann dabei über einen Abgleich mit der Bewegungsaktivität MOV sichergestellt werden, dass potentiell erhöhte Werte der Herzfrequenz HR ihrer Änderungsrate HRV nicht einer sportlichen Betätigung oder harter körperlicher Arbeit o.ä. geschuldet sind, sondern für das Stressniveau des Trägers hinreichend hohe Aussagekraft besitzen. Die so ermittelten Ruhewerte lassen sich zu einem späteren Zeitpunkt (nicht dargestellt) dazu verwenden, ein kritisches Stressniveau zu erkennen, um entsprechen das Unterprogramm 51 für die Signalverarbeitung anzuwenden, welches der akustischen Umgebungssituation 48 beim kritischen Stressniveau zugeordnet ist (anstatt dem Hörprogramm 50).

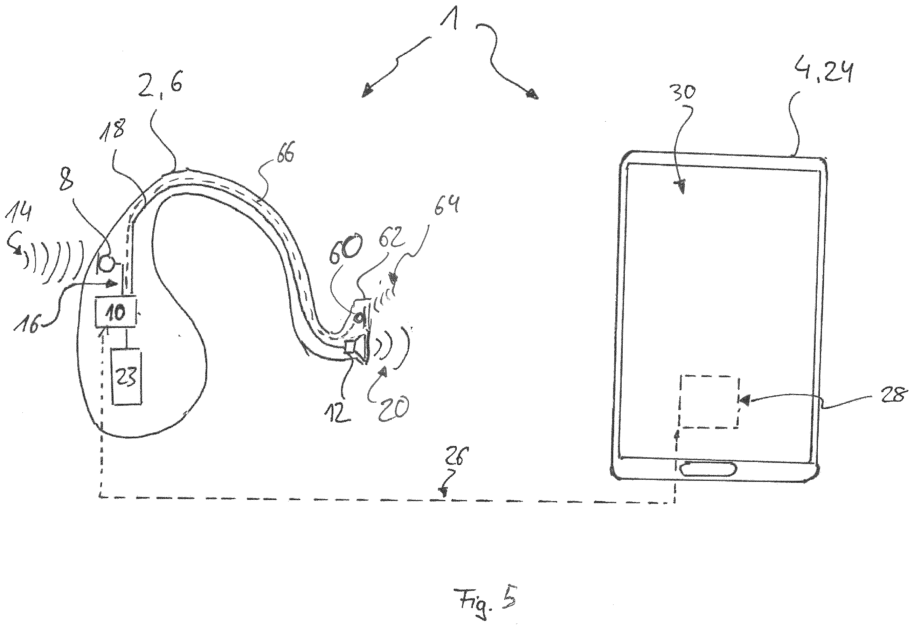

In Figur 5 ist schematisch in einem Blockschaltbild ein Hörsystem 1 dargestellt, welches ein Hörinstrument 2 aufweist, das eine Alternative zum in Figur 1dargestellten Hörinstrument 2 bildet. Das Hörinstrument 2 der Figur 5 weist anstatt des PPG-Sensors 22 ein Gehörgang-Mikrofon 60 auf (in einer weiter alternativen Variante kann das Hörinstrument auch den PPG-Senso 22 und das Gehörgang-Mikrofon 60 aufweisen), welches zusammen mit dem Lautsprecher 12 in einem Ohrstück 62 angeordnet ist. Das Ohrstück 62 ist für den bestimmungsgemäßen Betreib des Hörinstruments 2 vom Träger (nicht dargestellt) wenigstens teilweise in einen Eingang eines seiner beiden Gehörgänge einzuführen, sodass das Gehörgang-Mikrofon 60 dadurch in den Gehörgang gerichtet ist, und dort einen Gehörgangs-Schall 64 im durch das Ohrstück 62 wenigstens teilweise verschlossenen Gehörgang aufnehmen kann.

Das Gehörgang-Mikrofon 60 erzeugt aus dem Gehörgangs-Schall 64 ein zusätzliches Eingangssignal 66 (gestrichelte Linie), welches zur Signalverarbeitungseinrichtung 10 weitergeleitet wird. Aus dem Gehörgangs-Schall 64 kann mittels der darüber im zusätzlichen Eingangssignal 66 die Herzfrequenz durch eine entsprechende Analyse und/oder Filterung des zusätzlichen Eingangssignals 66 ermittelt werden. Die besagte Analyse bzw. Filterung des zusätzlichen Eingangssignals 66 kann hierbei insbesondere in der Signalverarbeitungseinrichtung 10 erfolgen. Das Gehörgang-Mikrofon 60 kann hierbei insbesondere noch eine weitere Funktionalität erfüllen, etwa im Rahmen einer Okklusionsunterdrückung, welche bevorzugt durch die Signalverarbeitungseinrichtung 10 angesteuert wird.

Die weitere Verarbeitung der anhand des zusätzlichen Eingangssignals 66 ermittelten Herzfrequenz verläuft wie oben bereits beschrieben.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

### Bezugszeichenliste

- 1: Hörsystem
- 2: Hörinstrument
- 4: Hilfsgerät
- 6: Hörgerät
- 8: Mikrofon
- 10: Signalverarbeitungseinrichtung
- 12: Lautsprecher
- 14: Umgebungsschall
- 16: Eingangssignal
- 18: Ausgangssignal
- 20: Ausgangsschallsignal
- 22: PPG-Sensor
- 23: Beschleunigungssensor
- 24: Smartphone
- 26: Bluetooth-Verbindung
- 28: Prozessoreinheit
- 30: Touchscreen
- 42: biometrische Parameter
- 43: Kenngröße einer kardiovaskulären Aktivität
- 44: Sprachanalyse
- 46: Benutzereingabe
- 47: Analyse
- 48: akustische Umgebungssituation
- 50: Hörprogramm
- 51: Unterprogramm
- 52: Parameterwert
- 54: Parameter (der Signalverarbeitung)
- 56: Benutzereingabe
- 58n/p: negative/positive Bewertung
- 60: Gehörgang-Mikrofon
- 62: Ohrstück
- 64: Gehörgangs-Schall
- 66: zusätzliches Eingangssignal

- CSL: kritisches Stressniveau
- FBs: Frequenzbänder mit hohen Sprachanteil
- gj: frequenzbandweiser Verstärkungsfaktor
- HR: Herzfrequenz
- HR-0: Ruhewert (der Herzfrequenz)
- HR-1: (kritischer) Wert (der Herzfrequenz)
- HRV: Änderungsrate (der Herzfrequenz)
- HRV-0: Ruhewert (der Änderungsrate)
- HRV-1: (kritischer) Wert (der Änderungsrate)
- MOV: Bewegungsaktivität
- n: "Nein"-Pfad (der Sprachanalyse)
- NR: Rauschunterdrückung
- Ons: Beginn von Sprachsegmenten
- Pmaxj: frequenzbandweiser maximaler Ausgangspegel
- Tatc: Einregelzeit (einer Kompression)
- Trls: Abklingzeit (einer Kompression)
- y: "Ja"-Pfad (der Sprachanalyse)

## Patentansprüche

1. Verfahren zum Betrieb eines Hörinstrumentes (2),
- mittels eines Sensors (22) ein biometrischer Parameter (42) eines Trägers des Hörinstrumentes (2) erfasst wird und/oder
- mittels wenigstens eines Mikrofons (8) des Hörinstrumentes (2) aus einem Umgebungsschall (14) ein elektrisches Eingangssignal (16) erzeugt wird, und anhand einer Analyse des Eingangssignals (16) das Vorliegen einer bestimmten akustischen Umgebungssituation (48) aus einer Mehrzahl an unterschiedlichen akustischen Umgebungssituationen erkannt wird,
- anhand des erfassten biometrischen Parameters (42) bzw. der erkannten akustischen Umgebungssituation (48) auf ein kritisches Stressniveau (CSL) des Trägers geschlossen wird, und daraufhin
- abhängig vom so erschlossenen kritischen Stressniveau (CLS) wenigstens ein Parameter (54) einer Signalverarbeitung des Hörinstrumentes (2) auf einen vorab festgelegten Parameterwert (52) eingestellt wird.

2. Verfahren nach Anspruch 1,
wobei als biometrischer Parameter (42) eine Kenngröße (43) einer kardiovaskulären Aktivität des Trägers erfasst wird.

3. Verfahren nach Anspruch 2,
wobei als Kenngröße einer kardiovaskulären Aktivität (43) des Trägers eine Herzfrequenz (HR) des Trägers und/oder eine Änderungsrate (HRV) der Herzfrequenz und/oder ein systolischer und/oder diastolischer Blutdruck des Trägers und/oder eine Dauer einer Anspannungsphase des Herzes erfasst wird.

4. Verfahren nach Anspruch 3,
wobei die Herzfrequenz (HR) bzw. der systolische bzw. diastolische Blutdruck mittels einer Photoplethysmographie gemessen wird.

5. Verfahren nach Anspruch 3,
wobei die Herzfrequenz (HR) bzw. der systolische bzw. diastolische Blutdruck mittels eines Gehörgang-Mikrofons () gemessen wird, welches beim bestimmungsgemäßen Tragen des Hörinstrumentes (2) in einen Gehörgang des Trägers gerichtet ist.

6. Verfahren nach einem der Ansprüche 2 bis 5,
wobei mittels eines Beschleunigungssensors (23) und/oder eines Bewegungssensors ein Bewegungszustand (MOV) des Trägers ermittelt wird, und
wobei für das kritische Stressniveau (CSL) auch der ermittelte Bewegungszustand (MOV) des Trägers herangezogen wird.

7. Verfahren nach einem der Ansprüche 2 bis 6,
wobei durch die Analyse des Eingangssignals (16) das Vorliegen einer bestimmten akustischen Umgebungssituation (48) erkannt wird, und wobei für das kritische Stressniveau (CLS) auch die erkannte akustische Umgebungssituation (48) herangezogen wird.

8. Verfahren nach einem der Ansprüche 2 bis 7,
wobei zunächst ein Ruhewert (HR-0, HRV-0) für die Kenngröße (43) der kardiovaskulären Aktivität ermittelt wird, und
wobei die erfasste Kenngröße (43) der kardiovaskulären Aktivität mit dem entsprechenden Ruhewert (HR-0, HRV-0) verglichen wird, und hieraus auf das kritisches Stressniveau (CSL) des Trägers geschlossen wird.

9. Verfahren nach Anspruch 8,
wobei zum Ermitteln des Ruhewertes (HR-0, HRV-0) jeweils
- der Bewegungszustand (MOV) des Trägers, welcher mittels des Beschleunigungssensors (23) bzw. des Bewegungssensors ermittelt wird, und/oder
- eine Tageszeit und/oder
- eine Kenngröße für einen Umgebungsschall (14), welche anhand einer entsprechenden Analyse des Eingangssignals (16) ermittelt wird, herangezogen werden.

10. Verfahren nach einem der Ansprüche 2 bis 9,
wobei anhand einer durch den Träger mittels einer Benutzerschnittstelle vorgenommenen Benutzereingabe (46) ein kritischer Wert (HR-1, HRV-1) für die Kenngröße der kardiovaskulären Aktivität ermittelt wird, welcher einem kritischen Stressniveau (CSL) des Trägers entspricht, und wobei zu einem späteren Zeitpunkt in Abhängigkeit des kritischen Wertes (HR-1, HRV-1) für die Kenngröße der kardiovaskulären Aktivität auf das kritische Stressniveau (CSL) geschlossen wird.

11. Verfahren nach einem der vorhergehenden Ansprüche,
wobei
- anhand des Eingangssignals (16) das Vorliegen einer bestimmten akustischen Umgebungssituation (48) aus der Mehrzahl an unterschiedlichen akustischen Umgebungssituationen erkannt wird,
- ein der bestimmten akustischen Umgebungssituation (48) vorab zugeordnetes Hörprogramm (50) für die Signalverarbeitung des Eingangssignals (16) angewandt wird, welches dem wenigstens einen Parameter (54) einen bestimmten Parameterwert (52) zuweist,
- anhand einer Benutzereingabe (56) des Trägers eine Bewertung (58n, 58p) des Hörprogramms (50) durch den Träger erfasst wird,
- bei einem Erfassen eines kritischen Stressniveaus (CSL) des Trägers
-- im Fall einer negativen Bewertung (58n) des Hörprogramms (50) die Gewichtung der negativen Bewertung (58n) bei einem Benutzer-assistierten, automatischen Anpassen des Parameterwertes (52) für die bestimmte akustische Umgebungssituation (48) verringert wird, und/oder
-- im Fall einer positiven Bewertung (58p) des Hörprogramms (50) aus dem Hörprogramm (50) ein den Parameterwert (52) umfassendes Unterprogramm (51) erzeugt wird, dessen Anwendung für die bestimmte akustische Umgebungssituation (48) bei einem kritischen Stressniveau (CSL) des Trägers erfolgt.

12. Verfahren nach einem der vorhergehenden Ansprüche,
wobei
- anhand des Eingangssignals (16) das Vorliegen einer bestimmten akustischen Umgebungssituation (48) aus der Mehrzahl an unterschiedlichen akustischen Umgebungssituationen erkannt wird,
- bei einem Erfassen eines kritischen Stressniveaus (CSL) des Trägers dieses der bestimmten akustischen Umgebungssituation (48) zugeordnet wird, und
wobei anhand der Analyse des Eingangssignals (16) das Vorliegen der bestimmten akustischen Umgebungssituation (48) erkannt wird, und anhand der erkannten akustischen Umgebungssituation (48) auf das kritisches Stressniveau (CSL) des Trägers geschlossen wird.

13. Verfahren nach einem der vorhergehenden Ansprüche,
wobei das Eingangssignal (16) auf ein Vorhandensein eines Sprachsignals um Umgebungsschall (14) hin analysiert wird,
wobei beim Vorhandensein eines Sprachsignals der wenigstens eine Parameter (54) der Signalverarbeitung derart auf den vorab festgelegten Parameterwert (52) eingestellt wird, dass
- eine Richtwirkung eines Richtmikrofons verstärkt wird, und/oder
- ein Beginn (Ons) von Sprachsegmenten hervorgehoben wird, und/oder
- Frequenzbänder (FBs) mit einem hohen Sprachanteil angehoben werden,
und/oder bei Abwesenheit eines Sprachsignals der Signalverarbeitung derart auf den vorab festgelegten Parameterwert (52) eingestellt wird, dass
- eine frequenzbandweise Verstärkung (gj) und/oder ein frequenzbandweiser maximaler Ausgangspegel (Pmaxj) abgesenkt werden, und/oder
- eine Einregelzeit (Tatc) einer Kompression verkürzt und/oder eine Abklingzeit (Trls) einer bzw. der Kompression verlängert werden, und/oder
- eine Anwendungsstärkte einer Rauschunterdrückung (NR) und/oder eines Glättungsfilters angehoben werden.

14. Verfahren nach einem der vorhergehenden Ansprüche,
wobei bei einem kritischen Stressniveau (CSL) eine Anwendungsstärke und/oder eine Notch-Tiefe eines Tinnitus-Maskierungssignals erhöht wird.

15. Hörsystem (1), umfassend
- ein Hörinstrument (2)
- einen Sensor (22) zum Erfassen eines biometrischen Parameters (42) eines Trägers des Hörinstrumentes (2), welcher Aufschluss über ein kritisches Stressniveau (CSL) des Trägers gibt, und
- eine Steuereinrichtung,
wobei die Steuereinrichtung dazu eingerichtet ist, das Hörinstrument (2) in Abhängigkeit des biometrischen Parameters (42) für das kritische Stressniveau (CSL) gemäß dem Verfahren nach einem der vorhergehenden Ansprüche zu betreiben.
